(19) Europäisches Patentamt
European Patent Office
Office européen des brevets

(11) EP 0 634 410 B1

(12) **EUROPÄISCHE PATENTSCHRIFT**

(45) Veröffentlichungstag und Bekanntmachung des Hinweises auf die Patenterteilung:
**26.02.1997 Patentblatt 1997/09**

(51) Int Cl.⁶: **C07D 401/06**, C07D 209/48, C07D 411/06

(21) Anmeldenummer: **94110247.7**

(22) Anmeldetag: **01.07.1994**

(54) **Verfahren zur Herstellung eines N-tert.Butylisochinolin-3-carboxamid Derivates und Zwischenprodukte in diesem Verfahren**

Process for the preparation of a N-tert.butylisoquinolin-3-carboxamide derivative and intermediate compounds of these process

Procédé de préparation d'un dérivé du N-tert.butyl isoquinoline-3-carboxamide et produits intermédiaire de ces procédés

(84) Benannte Vertragsstaaten:
**AT BE CH DE DK ES FR GB IT LI NL**

(30) Priorität: **15.07.1993 CH 2132/93**
**04.08.1993 CH 2330/93**

(43) Veröffentlichungstag der Anmeldung:
**18.01.1995 Patentblatt 1995/03**

(60) Teilanmeldung: **96112342.9**

(73) Patentinhaber: **F. HOFFMANN-LA ROCHE AG**
**4002 Basel (CH)**

(72) Erfinder: **Hilpert, Hans**
**CH-4153 Reinach (CH)**

(74) Vertreter: **Mahé, Jean et al**
**F.Hoffmann-La Roche AG**
**Patent Department (PLP),**
**124 Grenzacherstrasse**
**4070 Basel (CH)**

(56) Entgegenhaltungen:
**EP-A- 0 432 694**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 634 410 B1

**Beschreibung**

Die vorliegende Erfindung betrifft ein neues Verfahren zur Herstellung von N-tert. Butyl-decahydro-2-[2(R)-hydroxy-4-phenyl-3(S)-phthalimidobutyl]-(4aS,8aS)-isochinolin-3(S)-carboxamid der Formel

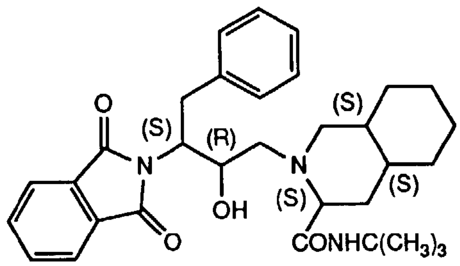

I

sowie neue Zwischenprodukte in dem genannten Verfahren.

Die Verbindung der obigen Formel I ist beispielsweise in Beispiel 1 der Europäischen Patentpublikation 0.432.694 spezifisch beschrieben und ist ein wertvolles Zwischenprodukt zur Herstellung von pharmakologisch aktiven Verbindungen. So kann die Verbindung der Formel I wie in den Beispielen 4 und 5 der genannten Europäischen Patentpublikation beschrieben in pharmakologisch aktive Verbindungen übergeführt werden, welche sich zur Behandlung viraler Infektionen eignen, insbesondere solcher Infektionen, die durch HIV und andere Retroviren verursacht werden.

Das erfindungsgemässe Verfahren ist dadurch gekennzeichnet, dass man (2S,3S)-3-(1,3-Dioxo-2,3-dihydro-1H-isoindol-2-yl)-2-hydroxy-4-phenylbutyraldehyd der Formel

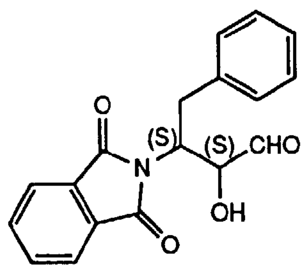

II

mit N-tert.Butyl-decahydro-(4aS,8aS)-isochinolin-3(S)-carboxamid der Formel

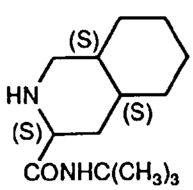

III

reduktiv aminiert.

Die reduktive Aminierung des Hydroxyaldehyds der Formel II mit der Verbindung der Formel III erfolgt in an sich bekannter Weise. So kann man sie in einem Lösungsmittel, wie Methanol, in Gegenwart von einem Alkalimetallborhydrid, z.B. einem Alkalimetallcyanborhydrid der Formel $MBH_3CN$, worin M ein Alkalimetall, wie Lithium oder Natrium ist, und einer Säure, wie Salzsäure, durchführen. In einer Variante wird eine Lösung der Verbindungen der Formeln II und III in einer Säure, wie Essigsäure, mit Lithiumcyanborhydrid oder Natriumcyanborhydrid versetzt. In einer weiteren Variante wird eine Lösung der Verbindungen der Formel II und III in Ameisensäure zum Rückfluss erhitzt.

Die in den obigen Verfahren als Ausgangsstoff eingesetzte Verbindung der Formel II ist neu und ebenfalls Gegenstand der vorliegenden Erfindung. Sie kann wie im nachstehenden Reaktionsschema formelmässig angegeben hergestellt werden. In dem Reaktionsschema bedeutet R nieder Alkyl oder Phenyl, das durch ein oder zwei Halogenatome substituiert sein kann.

Die als Ausgangsstoff eingesetzte Verbindung der Formel III ist bekannt und entspricht derjenigen der Formel VII

in der Europäischen Patentpublikation 0.432.695.

Der oben verwendete Ausdruck "nieder-Alkyl" betrifft geradkettige und verzweigte gesättigte Kohlenwasserstoffreste mit 1-6, vorzugsweise 1-4, Kohlenstoffatomen, wie Methyl, Aethyl, n-Propyl, Isopropyl, n-Butyl, Isobutyl, sec. Butyl, tert.Butyl, Pentyl, Hexyl und dergleichen. Der Ausdruck "Halogen" betrifft Fluor, Chlor, Brom und Jod.

Der (2S,3S)-3-(1,3-Dioxo-2,3-dihydro-1H-isoindol-2-yl)-2-hydroxy-4-phenylbutyraldehyd der Formel II kann entsprechend der Sequenz V→VIII→XI→II in Reaktionsschema hergestellt werden. Im ersten Schritt dieser Sequenz wird das 3-Phenyl-2(S)-phthalimidopropan-1-al der Formel V in an sich bekannter Weise in Gegenwart einer Base mit Nitromethan umgesetzt. Diese Umsetzung kann man zweckmässig in einem Lösungsmittel, wie Tetrahydrofuran und dergleichen, unter Abkühlen, z.B. auf -20° bis +5°C, vorzugsweise auf -15° bis 0°C, durchführen. Dabei verwendet man katalytische oder stöchiometrische Mengen einer Base, wie Kaliumtert.butylat, Na-Methylat oder Aluminiumoxid. Das erwünschte (1S,2R)-Isomere des erhaltenen Gemisches der (1S,2S)- und (1S,2R)-Isomeren des 2-(1-Benzyl-2-hydroxy-3-nitropropyl)-2,3-dihydro-1H-isoindol-1,3-dions der Formel VIII kann man durch Kristallisation, z.B. aus tert.-Butylmethyläther anreichern, oder durch Chromatographie, z.B. an Kieselgel mit Hexan/Äthylacetat, in reiner Form isolieren.

Das so erhaltene (1S,2R)-2-(1-Benzyl-2-hydroxy-3-nitropropyl)-2,3-dihydro-1H-isoindol-1,3-dion der Formel VIII wird im nächsten Schritt ebenfalls in an sich bekannter Weise in das (1S,2S)-2-(1-Benzyl-2-hydroxy-3,3-dimethoxypropyl)-2,3-dihydro-1H-isoindol-1,3-dion der Formel XI übergeführt, und zwar durch Umsetzen mit einem Alkalimetallalkoholat, wie Natriummethylat, und einer Säure, wie Schwefelsäure, Salzsäure oder Bromwasserstoffsäure. Diese Umsetzung wird vorzugsweise in einem Lösungsmittel, wie Methanol, unter Abkühlen, z.B. auf -50° bis +10°C, vorzugsweise auf -35°C, durchgeführt. Im letzten Schritt dieser Sequenz kann das so erhaltene Hydroxyacetal der Formel XI nach ebenfalls an sich bekannter Methode durch Säurebehandlung in den Aldehyd der Formel II übergeführt werden. Die Säurebehandlung erfolgt zweckmässigerweise in einem unter den Reaktionsbedingungen inerten organischen Lösungsmittel, wie einem Aether, z.B. Tetrahydrofuran oder Dioxan, und dergleichen bei Raumtemperatur. Als Säure kommt insbesondere eine Mineralsäure, wie Salzsäure oder Schwefelsäure, in Frage.

Die Verbindungen der Formeln II, VIII und XI sind neu und ebenfalls Gegenstand der vorliegenden Erfindung. Das 3-Phenyl-2(S)-phthalimidopropan-1-al der Formel V erhält man durch Erhitzen von L-Phenylalanin mit Phthalsäureanhydrid in Toluol, Umsetzung des entstandenen N-geschützten L-Phenylalanins mit Oxalylchlorid in Toluol und katalytischen Mengen Dimethylformamid und katalytischer (Pd/C) Hydrierung des entstandenen, dem erwünschten Aldehyd der Formel V entsprechenden Säurechlorids in Gegenwart von 1,2-Butylenoxid in Toluol.

Die folgenden Beispiele sollen die vorliegende Erfindung erläutern, sie jedoch in keiner Weise einschränken. Alle Temperaturen sind in Celsiusgraden angegeben.

## Reaktionsschema

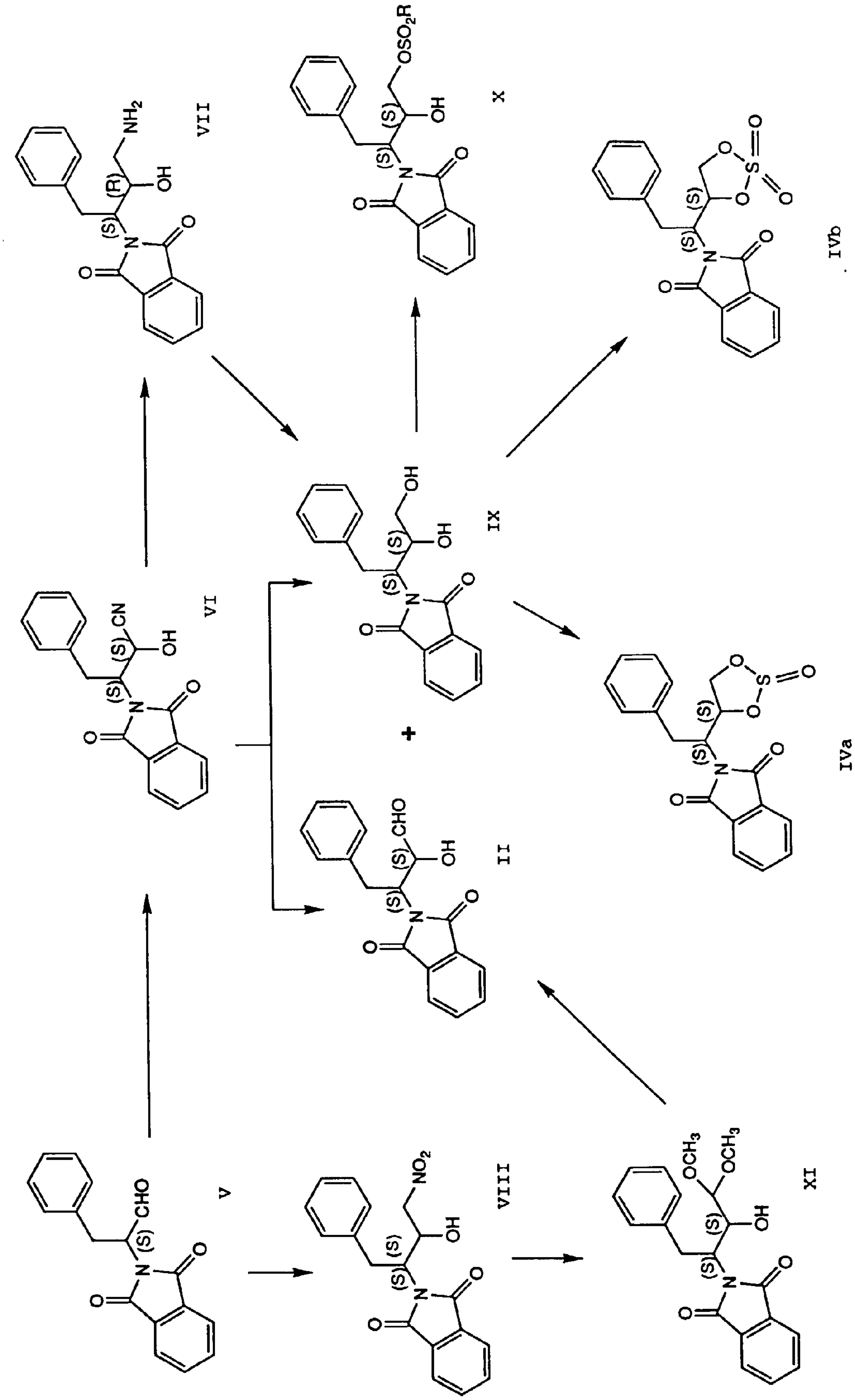

VII X IVb VI IX IVa II V VIII XI

Beispiel 1

A) Eine Suspension von 82,6 g L-Phenylalanin und 74,1 g Phthalsäureanhydrid in 600 ml Toluol wird 8 Stunden unter Argon zum Rückfluss erhitzt. Die entstandene Suspension wird auf Raumtemperatur abgekühlt und mit 0,5 ml Dimethylformamid, gefolgt von 66,64 g Oxalylchlorid, versetzt. Nach 2 Stunden Rühren wird Argon in die Suspension geblasen.

B) Die das 3-Phenyl-2(S)-phthalimidopropionylchlorid enthaltende Lösung wird mit 500 ml Toluol verdünnt und mit 72,11 g 1,2-Butylenoxid versetzt. Der Lösung werden 23,5 g Palladium auf Kohle (5%) und 100 ml Toluol zugesetzt. Die Suspension wird 17 Stunden unter Rühren hydriert und dann filtriert, und der Rückstand mit 200 ml Toluol gewaschen. Filtrat und Waschflüssigkeit werden vereinigt und unter Rühren mit einer Lösung von 0,5 Mol Natriumpyrosulfit in Wasser versetzt. Nach 4,5 Stunden werden die Phasen getrennt. Die wässrige Phase wird mit Toluol gewaschen. Die Toluolphasen werden mit Wasser gewaschen. Die vereinigten wässrigen Phasen werden mit Toluol und 3N Schwefelsäure versetzt und bei 60° gerührt. Danach werden die Phasen getrennt und die wässrige Phase mit Toluol extrahiert. Die Toluolphasen werden mit Wasser gewaschen, vereinigt, getrocknet und eingedampft. Man erhält das 3-Phenyl-2(S)-phthalimidopropan-1-al als weisser Feststoff, Schmelzpunkt 115-117°, $[\alpha]_D^{20}$ -200° (1% in Aethylacetat).

C) Eine Lösung von 20,0 g 3-Phenyl-2(S)-phthalimidopropan-1-al und 5,8 g Nitromethan in 120 ml Tetrahydrofuran wird unter Rühren bei -15° mit einer Lösung von 1,5 g Kalium-tert.butylat in 15 ml Tetrahydrofuran versetzt und 1 Stunde bei -15° und 1 Stunde bei 0° gerührt. Der pH-Wert der gelben Lösung wird mit 6 ml 3N Salzsäure auf 4 gestellt, und die Lösung mit 50 ml gesättigter Kochsalzlösung gewaschen. Die organische Phase wird getrocknet, filtriert, und das Filtrat zu 25 g (100%) eines 64:36-Gemisches von (1S,2R)-2-(1-Benzyl-2-hydroxy-3-nitropropyl) 2,3-dihydro-1H-isoindol-1,3-dion und (1S,2S)-2-(1-Benzyl-2-hydroxy-3-nitropropyl)-2,3-dihydro-1H-isoindol-1,3-dion eingeengt, IR(KBr):3550m (OH), 1766s und 1703s (C=O von Imid), 1556s und 1388s ($NO_2$). Aus dem Rohprodukt kann das (1S,2S)-Isomere durch Kristallisation aus tert.Butylmethyläther abgetrennt werden. Die beiden Isomeren lassen sich auch an Kieselgel mit Hexan/Aethylacetat (3:1) auftrennen.

D) Eine Suspension von 1,02 g eines 87:13-Gemisches der (1S,2R)- und (1S,2S)-Isomeren des 2-(1-Benzyl-2-hydroxy-3-nitropropyl)-2,3-dihydro-1H-isoindol-1,3-dions in 2 ml Methanol wird bei 0° mit 5,7 ml einer 7% Lösung von Natriummethylat in Methanol versetzt, und die entstandene gelbe Lösung zu einer auf -35° gekühlten Lösung von 7,2 ml Schwefelsäure in 27,6 ml Methanol während 20 Minuten getropft. Das Reaktionsgemisch wird anschliessend zu einem gerührten Gemisch von 150 ml Methylenchlorid und 70 ml Eiswasser gegeben, und die wässrige Phase mit Methylenchlorid extrahiert. Die vereinigten Methylenchlorid-Phasen werden getrocknet, filtriert und eingeengt, und der Rückstand mit Hexan/Aethylacetat (3:1) an Kieselgel gereinigt. Nach dem Eindampfen und Trocknen erhält man 0,52 g (49%) reines (1S,2S)-2-(1-Benzyl-2-hydroxy-3,3-dimethoxypropyl)-2,3-dihydro-1H-isoindol-1,3-dion als farbloses Oel, IR(Film): 3465m (OH), 1772s und 1708s (C=O von Imid), 1071s (C-O-C).

E) Eine Lösung von 107 mg (1S,2S)-2-(1-Benzyl-2-hydroxy-3,3-dimethoxypropyl)-2,3-dihydro-1H-isoindol-1,3-dion in 0,5 ml Tetrahydrofuran und 0,5 ml 3N Salzsäure wird 23 Stunden bei Raumtemperatur gerührt und danach vollständig eingeengt. Der Rückstand wird mit Hexan/Aethylacetat (2:1) an Kieselgel gereinigt. Nach dem Eindampfen und Trocknen erhält man 63 mg (68%) (2S,3S)-3-(1,3-Dioxo-2,3-dihydro-1H-isoindol-2-yl)-2-hydroxy-4-phenylbutyraldehyd als farblosen Schaum, MS:309.

F1) 309 mg (2S,3S)-3-(1,3-Dioxo-2,3-dihydro-1H-isoindol-2-yl)-2-hydroxy-4-phenylbutyraldehyd und 238 mg N-tert. Butyl-decahydro-(4aS,8aS)-isochinolin-3(S)-carboxamid werden in 5 ml Essigsäure gelöst und mit 120 mg Natriumcyanborhydrid portionsweise versetzt. Nach vollständigem Umsatz wird zur Trockene eingeengt, mit 10 ml Wasser versetzt und mit Methylenchlorid extrahiert. Die Extrakte werden getrocknet und filtriert, das Filtrat eingeengt, und der Rückstand mit Methylenchlorid/Methanol (95:5) an Kieselgel gereinigt. Man erhält das N-tert. Butyl-decahydro-2-[2(R)-hydroxy-4-phenyl-3(S)-phthalimidobutyl]-(4aS,8aS)-isochinolin-3(S)-carboxamid.

F2) In einer Variante zu Absatz F1) wird eine Lösung von (2S,3S)-3-(1,3-Dioxo-2,3-dihydro-1H-isoindol-2-yl)-2-hydroxy-4-phenylbutyraldehyd in einem Gemisch von 5 ml Methanol und 35 mg Chlorwasserstoff eingesetzt.

F3) In einer weiteren Variante zu Absatz F1) werden 309 mg (2S,3S)-3-(1,3-Dioxo-2,3-dihydro-1H-isoindol-2-yl)-2-hydroxy-4-phenylbutyraldehyd und 238 mg N-tert.Butyl-decahydro-(4aS,8aS)-isochinolin-3(S)-carboxamid in 5 ml Ameisensäure gelöst und bis zur vollständigen Umsetzung der Edukte auf 100° erhitzt . Die Aufarbeitung erfolgt wie unter Absatz F1) beschrieben.

Beispiel 2

Eine Suspension von 10,0 g eines 75:25-Gemisches der (2S,3S)- und (2R,3S)-Isomeren von 3-(1,3-Dioxo-2,3-dihydro-1H-isoindol-2-yl)-2-hydroxy-4-phenylbutyronitril, 2,7 ml 37%ige Salzsäure, 50 ml Isopropanol und 50 ml Wasser wird mit 1 g Palladium auf Kohle (10%) versetzt und 5 Stunden bei 100 kPa hydriert. Das Reaktionsgemisch wird filtriert, und das eingeengte Filtrat durch Chromatographie gereinigt, wobei ein Gemisch von (2S,3S)-3-(1,3-Dioxo-2,3-dihydro-1H-isoindol-2-yl)-2-hydroxy-4-phenylbutyraldehyd und (1S,2S)-2-(1-Benzyl-2,3-dihydroxypropyl)-2,3-dihydro-1H-isoindol-1,3-dion erhalten wird. IR (KBr) von (1S,2S)-(2S,3S)-3-(1,3-Dioxo-2,3-dihydro-1H-isoindol-2-yl)-2-hydroxy-4-phenylbutyraldehyd: 3451m (OH), 1774m und 1711s (C=O von Imid); IR von (1S,2S)-2-(1-Benzyl-2,3-dihydroxypropyl)-2,3-dihydro-1H-isoindol-1,3-dion: 3460m (OH).

Anstelle von 2,7 ml 37%iger Salzsäure kann auch 30 ml feuchter Ionenaustauscher (Dowex 50Wx4,H+) verwendet werden, wodurch bevorzugt (2S,3S)-3-(1,3-Dioxo-2,3-dihydro-1H-isoindol-2-yl)-2-hydroxy-4-phenylbutyraldehyd erhalten wird.

## Patentansprüche

1. Verfahren zur Herstellung von N-tert.Butyl-decahydro-2-[2(R)-hydroxy-4-phenyl-3(S)-phthalimidobutyl]-(4aS,8aS)-isochinolin-3(S)-carboxamid der Formel

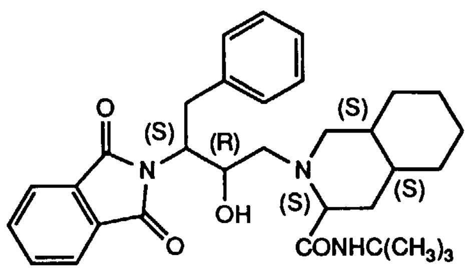

I

dadurch gekennzeichnet, dass man (2S,3S)-3-(1,3-Dioxo-2,3-dihydro-1H-isoindol-2-yl)-2-hydroxy-4-phenylbutyraldehyd der Formel

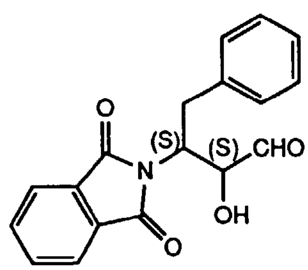

II

mit N-tert.Butyl-decahydro-(4aS,8aS)-isochinolin-3(S)-carboxamid der Formel

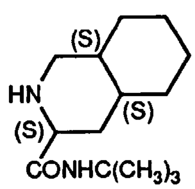

III

reduktiv aminiert.

2. (2S,3S)-3-(1,3-Dioxo-2,3-dihydro-1H-isoindol-2-yl)-2-hydroxy-4-phenylbutyraldehyd der Formel

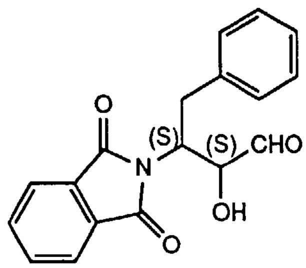

II

**3.** (1S,2R)-2-(1-Benzyl-2-hydroxy-3-nitropropyl)-2,3-dihydro-1H-isoindol-1,3-dion der Formel

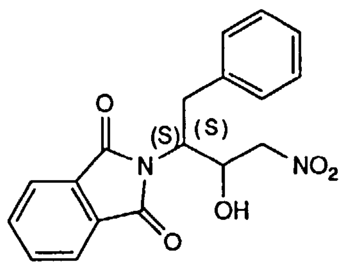

VIII

**4.** (1S,2S)-2-(1-Benzyl-2-hydroxy-3,3-dimethoxypropyl)-2,3-dihydro-1H-isoindol-1,3-dion der Formel

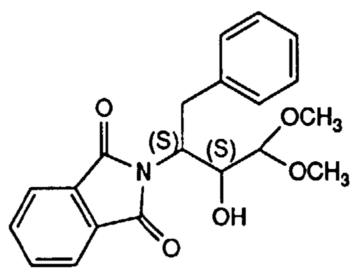

XI

## Claims

**1.** A process for the manufacture of N-tert.butyldecahydro-2-[2(R)-hydroxy-4-phenyl-3(S)-phthalimidobutyl]-(4aS, 8aS)-isoquinoline-3(S)-carboxamide of the formula

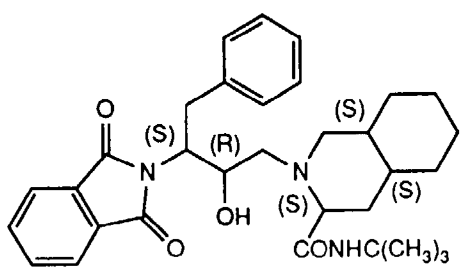

I

characterized by reductively aminating (2S,3S)-3-(1,3-dioxo-2,3-dihydro-1H-isoindol-2-yl)-2-hydroxy-4-phenylbutyraldehyde of the formula

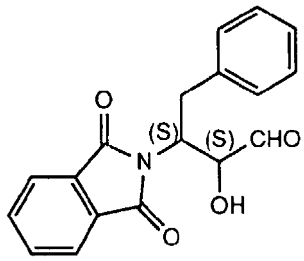

II

with N-tert.butyl-decahydro-(4aS,8aS)-isoquinoline-3(S)-carboxamide of the formula

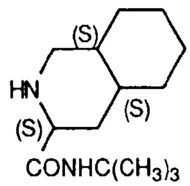

III.

2. (2S,3S)-3-(1,3-Dioxo-2,3-dihydro-1H-isoindol-2-yl)-2-hydroxy-4-phenylbutyraldehyde of the formula

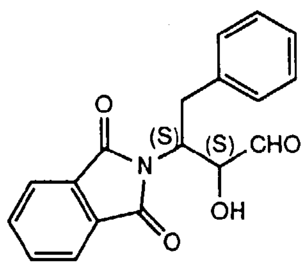

II

3. (1S,2R)-2-(1-Benzyl-2-hydroxy-3-nitropropyl)-2,3-dihydro-1H-isoindole-1,3-dione of the formula

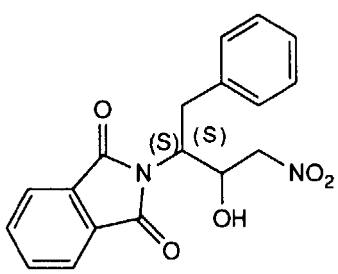

VIII

4. (1S,2S)-2-(1-Benzyl-2-hydroxy-3,3-dimethoxypropyl)-2,3-dihydro-1H-isoindole-1,3-dione of the formula

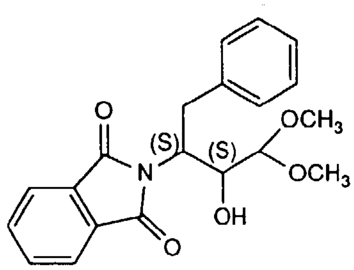

XI

## Revendications

1. Procédé de préparation de N-tert.butyl-décahydro-2-[2(R)-hydroxy-4-phényl-3(S)-phtalimidobutyl]-(4aS,8aS)-isoquinoléine-3(S)-carboxamide de formule

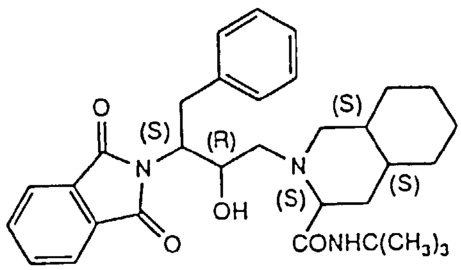

I

caractérisé en ce que
on effectue une amination réductrice du (2S,3S)-3-(1,3-dioxo-2,3-dihydro-1H-isoindol-2-yl)-2-hydroxy-4-phénylbutyraldéhyde de formule

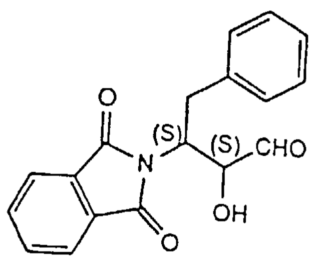

II

avec le N-tert.butyl-décahydro-(4aS,8aS)-isoquinoléine-3(S)-carboxamide de formule

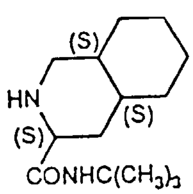

. III

2. (2S,3S)-3-(1,3-dioxo-2,3-dihydro-1H-isoindol-2-yl)-2-hydroxy-4-phényl-butyraldéhyde de formule

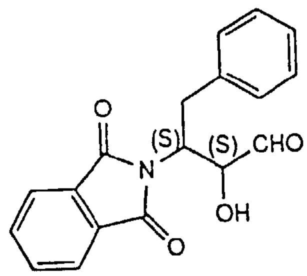

II

3. (1S,2R)-2-(1-benzyl-2-hydroxy-3-nitropropyl)-2,3-dihydro-1H-isoindole-1,3-dione de formule

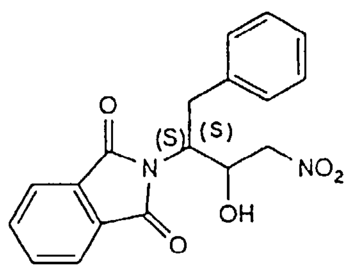

VIII

4. (1S,2S)-2-(1-benzyl-2-hydroxy-3,3-diméthoxypropyl)-2,3-dihydro-1H-isoindole-1,3-dione de formule

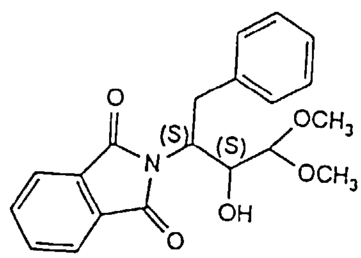

XI